# EUROPEAN PATENT APPLICATION

(11) **EP 3 118 313 A1**
(43) Date of publication of application: **18.01.2017**
(21) Application number: 15177361.1
(22) Date of filing: 17.07.2015
(51) Int. Cl.: C12N 15/10, C12Q 1/68

(54) **CLONING OF SINGLE-STRANDED RNA**

(71) Applicant: Max-Planck-Gesellschaft zur Förderung der Wissenschaften e.V., 80539 München (DE)
(72) Inventor: The designation of the inventor has not yet been filed
(74) Representative: Vossius & Partner Patentanwälte Rechtsanwälte mbB

(57) **Abstract**

The present invention relates to an oligonucleotide comprising (a) a double-stranded portion, which double-stranded portion is DNA and 9 to 30 base in length; (b) a loop connecting the 3' end of the first strand of said double-stranded portion with the 5' end of the second strand of said double-stranded portion, said loop comprising, in 5' to 3' direction: (ba) a first DNA portion which is 4 to 20 nucleotides in length; (bb) a non-nucleic acid spacer which (i) does not interfere with the formation of a stem-loop by said oligonucleotide; and (ii) causes polymerases to cease; and (bc) a second DNA portion which is 4 to 20 nucleotides in length; wherein said first DNA portion and said second DNA portion are not complementary to each other; (c) a single-stranded overhang at its 5' end, said overhang being 5 to 40 nucleotides in length, wherein (ca) the bond connecting said double-stranded portion with the nucleotide of said overhang which is directly adjacent to said double-stranded portion is cleavable under alkaline conditions; and (cb) said overhang optionally comprises a barcode sequence, said barcode sequence preferably being 5 to 10 nucleotides in length; and optionally (d) within one, more or all of (a), (b) and (c), one, more or all of the following: (da) one or more modified nucleotides; (db) one or more sequences conferring compatibility with nucleic acid sequencing kits, such compatibility being preferably the presence of regions within said oligonucleotide which are complementary to primers comprised in said sequence kits; and (dc) one or more random bases.

## Description

The present invention relates to an oligonucleotide comprising (a) a double-stranded portion, which double-stranded portion is DNA and 9 to 30 base in length; (b) a loop connecting the 3' end of the first strand of said double-stranded portion with the 5' end of the second strand of said double-stranded portion, said loop comprising, in 5' to 3' direction: (ba) a first DNA portion which is 4 to 20 nucleotides in length; (bb) a non-nucleic acid spacer which (i) does not interfere with the formation of a stem-loop by said oligonucleotide; and (ii) causes polymerases to cease; and (bc) a second DNA portion which is 4 to 20 nucleotides in length; wherein said first DNA portion and said second DNA portion are not complementary to each other; (c) a single-stranded overhang at its 5' end, said overhang being 5 to 40 nucleotides in length, wherein (ca) the bond connecting said double-stranded portion with the nucleotide of said overhang which is directly adjacent to said double-stranded portion is cleavable under alkaline conditions; and (cb) said overhang optionally comprises a barcode sequence, said barcode sequence preferably being 5 to 10 nucleotides in length; and optionally (d) within one, more or all of (a), (b) and (c), one, more or all of the following: (da) one or more modified nucleotides; (db) one or more sequences conferring compatibility with nucleic acid sequencing kits, such compatibility being preferably the presence of regions within said oligonucleotide which are complementary to primers comprised in said sequence kits; and (dc) one or more random bases.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Various methods to study protein-DNA interaction in living cells are available. These methods generally use formaldehyde cross-linking to fix DNA-protein interactions *in vivo,* after which the cells are sonicated to release pieces of chromatin which contain DNA fragments of a size in the range of ∼150-500 bp. DNA is then isolated, cloned using commercially available kits and sequenced. This is now considered a standard practice and results of numerous such experiments are publicly available.

Studying protein-RNA interactions is more challenging. Even though there are a few protocols, these methods are not widely applied because they are labor-intensive and require the use of radioactivity.

In more detail, an established protocol typically involves: (i) cross-linking of the protein of interest to RNA with UV-C or a chemical cross-linker such as formaldehyde; (ii) immunoprecipitating the protein with a specific antibody; (iii) isolating bound RNA; (iv) cloning of the ssRNA with a commercially available kit; (v) sequencing; and (vi) analyzing the sequences determined.

The resulting data is usually extremely convoluted due to the presence of contaminating RNA such as tRNAs, snRNAs, ribosomal RNAs etc. Secondly, the sequenced fragments will have a broad profile, i.e. most RNA-binding proteins will recognize 6-12 nts, but the fragments that are sequenced will be ∼100-200 nt without any positional information as to where the binding of the RNA-binding proteins might have happened in the cell.

More recent protocols (CLIP, HITS-CLIP, PAR-CLIP, iCLIP, CRAC, CLASH) circumvent these shortcomings. At the end of the protocol, often relatively clean data (much less contamination from tRNAs, snRNAs, ribosomal RNAs) are obtained. Also, the position of the cross-linking site may be determined. Drawbacks include that these protocols are difficult, especially to the extent they rely on the use of radioactivity (γ-³²P ATP is used to mark bound RNAs); and generally they take about a week to be completed.

WO2012/103154 describes means and methods of manipulating nucleic acid molecules, especially by amplification and adaptor ligation. The document describes RNA/DNA chimeric stem/loop adaptor/primers which are utilized during target nucleic acid manipulation. The document does not address the issue of library generation from single-stranded nucleic acid.

Given the shortcomings of the prior art, the technical problem underlying the present invention can be seen in the provision of alternative or improved means and methods of sequencing single-stranded nucleic acids, of producing libraries from single-stranded nucleic acids and of analyzing protein-nucleic acid interactions.

The solution to these technical problems is provided by the aspects and embodiments disclosed below.

In a first aspect, the present invention provides an oligonucleotide comprising (a) a double-stranded portion, which double-stranded portion is DNA and 9 to 30 base in length; (b) a loop connecting the 3' end of the first strand of said double-stranded portion with the 5' end of the second strand of said double-stranded portion, said loop comprising, in 5' to 3' direction: (ba) a first DNA portion which is 4 to 20 nucleotides in length; (bb) a non-nucleic acid spacer which (i) does not interfere with the formation of a stem-loop by said oligonucleotide; and (ii) causes polymerases to cease; and (bc) a second DNA portion which is 4 to 20 nucleotides in length; wherein said first DNA portion and said second DNA portion are not complementary to each other; (c) a single-stranded overhang at its 5' end, said overhang being 5 to 40 nucleotides in length, wherein (ca) the bond connecting said double-stranded portion with the nucleotide of said overhang which is directly adjacent to said double-stranded portion is cleavable under alkaline conditions; and (cb) said overhang optionally comprises a barcode sequence, said barcode sequence preferably being 5 to 10 nucleotides in length; and optionally (d) within one, more or all of (a), (b) and (c), one, more or all of the following: (da) one or more modified nucleotides; (db) one or more sequences conferring compatibility with nucleic acid sequencing kits, such compatibility being preferably the presence of regions within said oligonucleotide which are complementary to primers comprised in said sequence kits; and (dc) one or more random bases.

The term "oligonucleotide" has its art-established meaning. It refers to a polycondensate of nucleotides. Nucleotides may be either ribonucleotides, deoxyribonucleotides and modified nucleotides. Modified nucleotides, the presence of which is optional, may be locked nucleotides. The use of locked nucleotides is a means of stabilizing the double-stranded portion.

A requirement of a certain part of the oligonucleotide of the invention to be DNA implies that this part consists exclusively of deoxyribonucleotides or of deoxyribonucleotides and modified nucleotides. The presence of ribonucleotides is excluded.

Similarly, to the extent a part of the oligonucleotide is required to be RNA (this applies to a preferred embodiment disclosed below), this implies that this part either consists exclusively of ribonucleotides or of ribonucleotides and modified nucleotides. Also in this context, preference is given to modified nucleotides which are locked nucleotides.

Preferably, the presence of modified nucleotides does not exceed 25%, 20%, 15%, 10%, 5%, 2% or 1% of the positions in the entire molecule and/or within anyone of (a), (ba), (bc) and (c).

Further envisaged modified nucleotides are nucleotides with modified bases and/or modifications of the ribose and/or modifications of the phosphate. Preferred modifications of the ribose include 2'-modifications such as 2'-O-methyl. Preferred modifications of the phosphate include thiophosphate.

In view of the definitions given above, an oligonucleotide according to the invention may comprise up to 140 nucleotides. For the ease of referencing, also for molecules of that length the term "oligonucleotide" is used herein. The minimal length, counting only nucleotides and not the non-nucleic acid spacer, is 31 nucleotides. The exemplary oligonucleotide disclosed further below and depicted in the figures has a length of 50 nucleotides.

The oligonucleotide according to the invention has three compulsory components, designated (a), (b) and (c).

The double-stranded portion in accordance with (a) is DNA in the sense of the definition given above. It may have length of 9, 10, 11, 12, 13, 14, 15, 16, 17, 18, 19, 20, 21, 22, 23, 24, 25, 26, 27, 28, 29 or 30 base pairs. Preference is given to perfect complementarity. Alternatively, one or more mismatches, for example one, two or three mismatches may be present. In either case the total number of nucleotides comprised in the double-stranded portion (a) is an even number between 18 and 60. The presence of bulges, preferably not more than 1, 2 or 3, while not being preferred, is not excluded. Given that a bulge results in non-identity of the numbers of nucleotides comprised in the first and second strand, respectively, it follows that the double-stranded portion may comprise a total number of nucleotides which is any integer value from 18 to 60.

The double-stranded portion, when considered in isolation, can be viewed as consisting of a first and a second strand. The first strand is the strand bearing the 5' overhang in accordance with feature (c). The second strand is the complementary strand.

The loop in accordance with (b) contains both DNA and a non-nucleic acid spacer. The two DNA portions in accordance with (ba) and (bc) each and independently of each other have a length of between 4 and 20 nucleotides, more preferably between 5 and 10 nucleotides. Any other integer value falling in these intervals, including 6, 7, 8, 9, 11, 12, 13, 14, 15, 16, 17, 18 and 19 is also envisaged. First and second DNA portions (ba) and (bc) may have equal or different length.

The non-nucleic acid spacer is defined in functional terms in the broadest embodiment. The chemistry is not particularly limited. The requirement "does not interfere with the formation of a stem loop by said oligonucleotide" implies a certain degree of flexibility. In particular, any structural implementation which would not permit base-pairing of the two strands of the double-stranded portion in accordance with (a) is excluded. Preferred implementations are the subject of embodiments disclosed below.

Different from the two strands forming the double-stranded portion in accordance with (a), the first and second DNA portions in accordance with (ba) and (bc) are not complementary to each other. Preferably, the term "not complementary" relates to the absence of any complementarity between first and second DNA portions in accordance with (ba) and (bc). This does not exclude, though, that in a less preferred embodiment there may be 1, 2 or 3 base pairs between first and second DNA portions in accordance with (ba) and (bc).

In a preferred embodiment, the following applies. For the purpose of this preferred embodiment, two hypothetical fully complementary double-stranded molecules are considered. The first molecule consists of (i) a strand which in turn consists of the first strand of said double-stranded portion (a) and the adjacent DNA portion (ba), and (ii) a strand which is perfectly complementary thereto. Analogously, the second hypothetical fully double-stranded molecule consists of (iii) a strand which in turn consists of the second DNA portion (bc) and the adjacent second strand of the double-stranded portion (a), and (iv) a strand which is perfectly complementary thereto. It is understood that while in these two hypothetical molecules, each of (ba) and (bc) have a complementary sequence, this does not apply to the oligonucleotide of the invention. The melting temperature as of each of the two hypothetical fully double-stranded molecules is determined or calculated. According to the mentioned preferred embodiment, the difference in melting temperature between the two hypothetical molecules shall not exceed 5°C. This is a means of optimizing amplification by PCR.

Constituent (c) has a single-stranded overhang which is 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 nucleotides in length. The only compulsory feature of said overhang is defined by feature (ca). A preferred structural implementation of requirement (ca), namely the first nucleotide at the 3' end of the 5' overhang being a ribonucleotide, is described further below.

A purpose of the oligonucleotide of the invention is to serve as an adaptor in the generation of a library, preferably a sequencing library. A sequencing library is a library designed for sequencing. State of the art adaptors are typically designed for nucleic acid material which is double-stranded, especially double-stranded DNA. The oligonucleotide according to the present invention is equipped with structural features which makes it useful for the generation of libraries from single-stranded nucleic acid. The term "single-stranded nucleic acid" includes single-stranded DNA as well as single-stranded RNA, single-stranded RNA being preferred. A common single-stranded RNA is mRNA. As such, the oligonucleotide of the present invention is particularly useful for experiments which aim at elucidating protein-single-stranded RNA interactions, especially protein-mRNA interactions. The term "elucidating" in this context includes determining the type of interaction, more specifically the sequence of the single-stranded RNA interacting with a given protein and/or the specific site(s) of interaction between said protein and said single-stranded RNA.

Deviating from state of the art adaptors, the oligonucleotide of the present invention is characterized *inter alia* by the following features: (i) The presence of a 5' overhang, i.e. feature (c) of the first aspect. This allows for reverse transcription of the construct formed by ligating single-stranded nucleic acid to the oligonucleotide of the invention (for further details see below). (ii) While the state of the art adaptors typically comprise two separate single strands which associate non-covalently, the adaptor of the present invention is one single-stranded oligonucleotide (which contains a double-stranded portion as defined above). As a consequence, the oligonucleotide of the invention assumes the structure of a stem loop. The loop portion of the stem loop contains nucleic acid elements as well as (iii) a non-nucleic acid spacer. The non-nucleic acid spacer causes polymerases to cease. As a consequence, in the protocol making use of the oligonucleotide as disclosed further below, there is no requirement for linearization prior to amplification by PCR.

As compared to the cumbersome protocols known from the prior art for the purpose of elucidating protein single-stranded nucleic acid interactions, the oligonucleotice of the present invention enables a robust, quick and high-throughput capable procedure. This procedure is the subject of further aspects of the present invention which are disclosed below.

Turning to the optional features of the oligonucleotide in accordance with the first aspect, it is of note that for certain applications multiplexing is desirable. Multiplexing means that nucleic acid, in particular single-stranded nucleic acid, originating from a plurality of sources is pooled. The pooled material is than subjected to downstream processing, downstream processing typically including sequencing. This is a means of further increasing throughput. On the other hand, the need arises to determine, upon sequencing, from which particular source a sequence of interest was originally derived from. This is done by using barcode sequences, wherein each source is given a different barcode. Barcode sequences are short oligonucleotide sequences having a length of preferably 5, 6, 7, 8, 9 or 10 nucleotides. The barcode sequences, if present, typically occur at a known position with the oligonucleotide.

The oligonucleotide according to the first aspect may furthermore comprise, within one, more or all of (a), (b) and (c), one or more optional features which are disclosed above and will be explained further below. These optional features do not entail the addition of nucleotides or sequences. Rather, they define how one, more or all of (a), (b) and (c) may be implemented.

The above phrase "one, more or all of (a), (b) and (c)" indicates that, for example, one or more modified nucleotides may be present only within (a), only within (b), only within (c), within (a) and (b), within (a) and (c), within (b) and (c), or within all of (a), (b) and (c). The same applies *mutatis mutandis* for the compatibility conferring sequences in accordance with (db). For example, and this will become apparent also in view of preferred or exemplary embodiments disclosed below, the sequence of (a) plus (ba) may comprise or consist of a sequence which is complementary to an art-established primer. Similarly, the sequence of (a) plus (bb) may comprise or consist of a sequence which is complementary to another art-established primer.

A further optional feature is the presence of one or more sequences in said oligonucleotide which one or more sequences confer compatibility to nucleic acid sequencing kits. Nucleic acid sequencing kits are well-established on the market and can be obtained from a number of manufacturers such as Illumina (San Diego, US) and Life Technologies (Carlsbad (CA), US; product name: IonTorrent). More specifically, the particular sequences chosen to implement the requirements laid down in the first aspect may be designed in a way that commercially available primers, e.g. from Illumina, recognize and bind to them. Such primers in turn may contain further features, in particular further sequences which are not comprised in the oligonucleotide according to the invention nor have a counterpart in the oligonucleotide according to the invention, which further sequences provide for binding to the oligonucleotide lawn present on the surface, e.g., of Illumina flow cells.

The same applies *mutatis mutandis* for compatibility with the IonTorrent system. Also this can be done by choosing the sequences in accordance with the above items (a), (ba) and (bc) appropriately.

To explain the feature of compatibility with sequencing kits further, we direct the attention to the exemplary constructs displayed in the figures enclosed herewith. In particular, the sequences of (a) plus (ba) of the construct shown in the figures together comprise a sequence which is complementary to a primer sequence designated "TruSeq Read 1" of the Ilumina system. This is the standard primer to sequence the 5' ends of libraries. However, this is not mandatory for sequencing or cluster generation in Illumina flow cells. One is in principle free to change (a) and (ba); this would simply entail the requirement to use a different primer for subsequent sequencing. Similarly, the sequences of (a) plus (bb) of the construct shown in the figures together comprise a sequence which is complementary to another standard Illumina sequencing primer ("Multiplex Read 2"). This primer is commonly used to sequences 3' end of libraries. "Complementary" in this context preferably means 100% complementarity, but allows for 1, 2 or 3 mismatches.

Compatibility with the commercially available sequencing kits does not entail a requirement to adapt the overhang in accordance with (c). This overhang is a special feature in accordance with the present invention which renders the oligonucleotide reverse-transcription capable.

A further optional feature are one or more, preferably 1, 2, 3, 4, 5, 6, 7, 8, 9 or 10, random bases. Preferably, said random bases are located in the 5' overhang (c). The term "random bases" designates a base with undetermined chemical nature. More specifically, a random base may be any one of A, C, G or T/U. A random base may occur within a ribonucleotide or a deoxyribonucleotide.

Random bases may be used for quality control, more specifically to differentiate between true PCR duplicates and sequence reads that seem to be PCR duplicates but actually are derived from independent ligation events.

A preferred position of a random base is the 5' end of the oligonucleotide according to the invention.

In a preferred embodiment one, more or all of said random bases may be semi-random bases. A semi-random base is a base which may be chosen from less than four of the naturally occurring bases in either DNA or RNA. Preferred semi-random bases are pyrimidines (Y) and purines (R). Semi-random bases Y are accordingly to be chosen from C and T/U, whereas semi-random bases R are to be chosen from A and G.

The alkaline conditions in accordance with (ca) preferably involve a pH above 12.

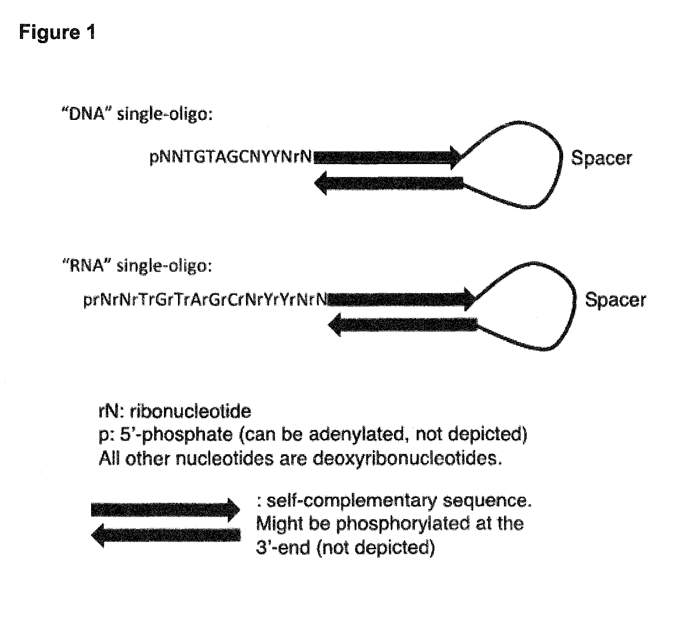

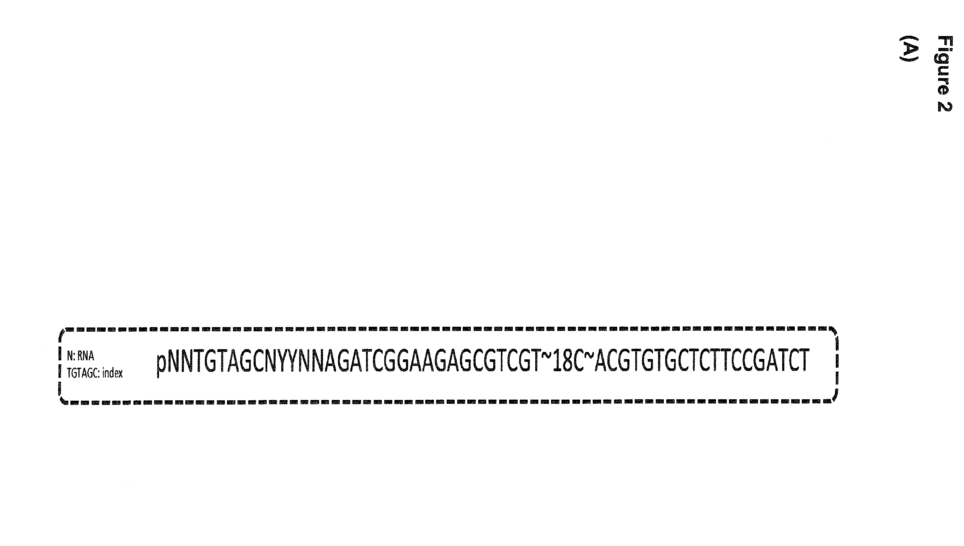

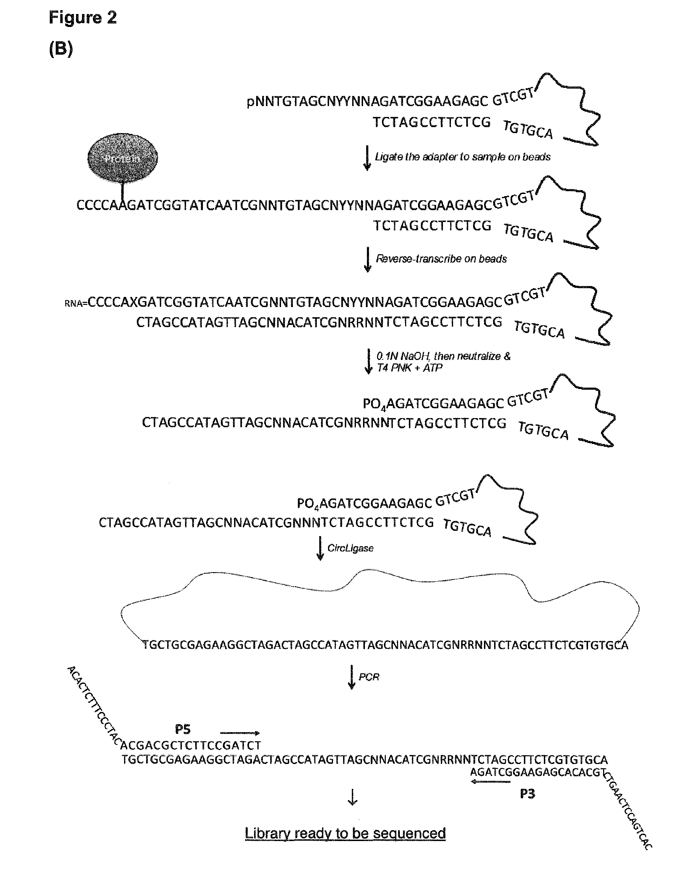

An exemplary oligonucleotide according to the invention is shown in part (A) of Figure 2. A schematic drawing illustrating key structural features is shown in Figure 1. As noted above, the single-stranded overhang (c) may be DNA or RNA. The two alternatives are shown in Figure 1. The nucleotide at the 3' end of the overhang which is the nucleotide of said overhang which is directly adjacent to the double-stranded portion (a) is a ribonucleotide in either case. The 5' nucleotide of the overhang (which is also the 5' end of the entire oligonucleotide) is phosphorylated. Positions 3 to 8 is an exemplary barcode sequence. The non-nucleic acid spacer in accordance with the present invention is shown as a curvy line in Figure 1 as well as in part (B) of Figure 2. In part (A) of Figure 2, it is indicated as "∼18C∼".

In a preferred embodiment, (a) the melting temperature of said double-stranded portion is between 37°C and 70°C, preferably between 60°C and 65°C; and/or (b) the number of mismatches between said first strand and said second strand is 0, 1, 2, or 3.

In a further preferred embodiment, said non-nucleic acid spacer is an oligo-ethyleneglycol spacer, preferably -(CH₂CH₂O)ₙ-, n being an integer number between 3 and 10, preferably 6. In other words, the phosphate connecting two adjacent nucleotides is preferably replaced by -O-(CH₂CH₂O)ₙ-OPO₃-.

In an alternative preferred embodiment, said non-nucleic acid spacer is an oligo-methylene spacer, preferably -(CH₂)ₘ-, m being an integer number between 5 and 50, 18 being a preferred value of m.

In a further preferred embodiment, said nucleotide of said overhang which is directly adjacent to said double-stranded portion comprises ribose.

In a further preferred embodiment, said overhang is RNA. To the extent modified nucleotides are comprised in said overhang, it is understood that it is also preferred that the backbone chemical bonds connecting any of said modified nucleotides with both upstream and downstream parts within said overhang are amenable to cleavage under those conditions where RNA is amenable to cleavage. Preferred cleavage conditions are disclosed herein below.

In the alternative, the overhang may be DNA or may contain both ribonucleotides and deoxyribonucleotides. Moreover, and as stated above, the option of modified nucleotides applies for the entire molecule. In any case, also when the overhang is made of DNA, feature (ca) of the first aspect in its broadest definition has to be met, wherein choosing said nucleotide of said overhang which is directly adjacent to said double-stranded portion to be a ribonucleotide is one option to implement feature (ca).

In a further preferred embodiment, (a) the 5' end of said oligonucleotide is amenable to ligation and/or comprises a 5' phosphate, a 5' hydroxy group, a 5' tri-phosphate or a 5' pre-adenylated phosphate; and/or (b) the 3' end of said oligonucleotide is capable of functioning as a primer and/or comprises a 3' hydroxy or a 3' phosphate.

The 3' end of the oligonucleotide is the starting point for reverse transcription.

In a further preferred embodiment, said one or more modified nucleotides, to the extent they are present, include LNA.

The term "LNA" designates locked nucleic acid. Locked nucleic acid as well as its building blocks, locked nucleotides, are known in the art. A methylene bridge connects 2' O and 4' C of the ribose. Locked nucleotides are a means of stabilizing the helical confirmation of double-stranded portions. Accordingly, locked nucleotides can be used to shorten double-stranded portions while maintaining a given melting temperature.

In a second aspect, the present invention provides the use of the oligonucleotide as defined in any one of the preceding aspects and embodiments for the generation of a library, preferably a sequencing library, from single-stranded nucleic acid.

In a preferred embodiment of said use, said generation involves reverse transcription and amplification. Preferred means and methods for implementing reverse transcription as well as amplification are disclosed further below.

In a further preferred embodiment of said use, said oligonucleotide and primers for said amplification are the only oligonucleotides to be used.

As noted above, a salient feature of the oligonucleotide according to the invention is that it contains sites for reverse transcription as well as binding sites for primers, said primers to be used for amplification. As a consequence, the oligonucleotide according to the invention is the only adaptor molecule required for generating libraries from single-stranded nucleic acid.

In a third aspect, the present invention provides a method of producing a library, preferably a sequencing library, from single-stranded RNA and/or single-stranded DNA, said method comprising or consisting of: (a) ligating said single-stranded RNA and/or single-stranded DNA to the oligonucleotide according to the first aspect; (b) reverse transcribing or transcribing, respectively, said single-stranded RNA or single-stranded DNA; (c) hydrolyzing any RNA; (d) circularizing the remaining DNA; and (e) subjecting the product of (d) to amplification by PCR.

This method yields a library which is amplified and ready for sequencing. In principle, sequencing can be effected by any means. To the extent the above described optional compatibility feature is used to implement complementarity with primers of a certain sequencing kit, it is obviously preferred to perform the downstream sequencing step with that particular kit.

Ligating in accordance with (a) can be effected with any ligase. Preferred is the use of T4 RNA ligase 1 in which case preference would be given to 5' phosphorylated oligos. Alternatively, it is possible to use T4 RNA ligase 2 (truncated form, preferably the KQ mutant) in which case preference is given to a pre-adenylated oligo. Pre-adenylated oligos are also compatible with T4 RNA ligase 1. Finally, RtcB ligase may be used, preferably in conjunction with a 5' OH oligonucleotide.

During ligation, said single-stranded RNA or single-stranded DNA is preferably bound to beads. Considering the preferred upstream pre-processing as detailed below, beads may e.g. be introduced in the course of step (2) of said pre-processing which step is purifying of a given protein. In the course of said purifying, use may be made of beads.

After ligating, one may conveniently proceed to reverse transcribing or transcribing, respectively, in accordance with (b). There is no need to add and anneal an oligonucleotide to prime a reverse transcription. This advantageous property is inherent to the ribonucleotide according to the first aspect of the present invention.

For the purpose of hydrolyzing in accordance with (c), any means which does not destroy the non-RNA part of the nucleic acid molecule generated in (b) may be used. Preferred means are discussed further below.

Circularizing in accordance with (b) is preferably done with CircLigase or CircLigase II.

Finally, amplification in accordance with (e) can be effected without any further purification or linearization. This is made possible by the non-nucleic acid spacer which causes the polymerase to cease.

In a preferred embodiment of the method according to the third aspect, said method comprises one or more of the following further steps: (aa) removing excess oligonucleotide after step (a) and prior to step (b); (ab) pooling of different samples containing ligation products obtained in (a), wherein ligation products originating from different samples are barcoded differently in accordance with feature (c)(cb) of the above disclosed first aspect, and wherein said step (ab) to be effected after step (a) and prior to step (b); (ac) removing, after step (a) and prior to step (b), the 3' phosphate from said ligation products to the extent it is present; and (ca) removing protein and small molecules from single-stranded DNA obtained in (c), step (ca) to be effected after step (c) and prior to step (d).

A preferred order of steps (aa), (ab) and (ac) is the following temporal order: (aa), followed by (ab), and then (ac).

Step (ab) provides for the above mentioned multiplexing.

Removal of the 3' phosphate in accordance to (ac) is preferred in order to prevent self-ligation. In principle, any phosphatase can be used for said purpose. T4 phosphonucleotide kinase (T4 PNK), preferably at a pH of about 6, can be used for that purpose. Any other phosphatase such as CIP, SAP, Antarctic phosphatase, FastAP, under the conditions specified by manufacturers, may be used in the alternative.

In a further preferred embodiment of the method of the invention, said hydrolyzing any RNA is effected, (c1) if the overhang of said oligonucleotide is RNA, by adding RNaseH; or (c2) if said overhang contains DNA, by adding 0.1 M NaOH.

Routinely, incubation with RNAseH is done at 37°C for 10 to 60 minutes.

For the removal of protein and small molecules in accordance with (ca), use can be made of the art-established means and methods including phenol/chlorophorm extraction followed by ethanol precipitation, silica or glass fibre columns suitable for DNA purification, or Solid Phase Reversible Immobilization (SPRI) beads.

Treatment with 0.1 M NaOH in accordance with (c2) is preferably done at a temperature between 37°C and 70°C, preferably at 70°C, for about 10 minutes. This will yield a hydroxy group at the 5' end of the remaining DNA molecule. This may be converted into a phosphate by using T4 PNK at neutral or slightly basic pH (pH 7 to 8) in the presence of ATP.

The method of generating a library as described so far may optionally be combined with an upstream pre-processing. This is the subject of the following preferred embodiment.

Accordingly, in a further preferred embodiment of the method in accordance with the third aspect, said method comprises the following steps prior to step (a): (1) optionally cross-linking a sample comprising cells; (2) purifying from said sample a given protein, said protein being bound or cross-linked to single-stranded or double-stranded nucleic acid; (3) converting said nucleic acid, if it is double-stranded, into single-stranded nucleic acid; and (4) trimming of the single-stranded nucleic acid bound or cross-linked to said protein.

Converting double-stranded DNA into single-stranded DNA may be effected with lambda exonuclease.

Trimming in accordance with step (4) is preferably done with RNAses. Useful enzymes include RNase I, RNase A, RNase V1, RNase T1, MNase and benzonase.

Given that RNase enzymes may leave a 2',3'-cyclic phosphate, a 3' phosphate or a 2' phosphate at the 3' end, further modification will be needed in order to generate an end which is suitable for ligation. Any phosphatase such as CIP, SAP, antarctic phosphatase, FastAP and T4 PNK may be used. Preference is given to T4 PNK at a pH of about 6.

In a particularly preferred embodiment of the above disclosed preferred embodiment, (1) cross-linking is effected by (1.1) UV-C; (1.2) UV-A after feeding said cells 4-thiouridine; or (1.3) formaldehyde; (2) purifying is effected by using (2.1) tandem affinity purification, in which case cross-linking is compulsory; (2.2) an antibody specific for said protein; and/or (3) trimming of said single-stranded nucleic acid, to the extent it is RNA, is effected with an RNase and/or by sonication.

As regards purifying, preference is given to tandem affinity purification. In that respect it is preferred to exploit the biotin/streptavidin interaction in one of the two steps. Avidin, NeutrAvidin or any suitable streptavidin derivative may be used as well.

In a further particularly preferred embodiment, said method comprises the following further steps after step (e): (f) sequencing one, more or all members of the library obtained in step (e); and (g) determining the most 5' nucleotide and/or the most 3' nucleotide of said nucleic acid bound or cross-linked to said protein, thereby determining the binding site of said protein on said nucleic acid.

Assuming that the site where the polymerase ceases is to be interpreted as the binding site of the RNA binding protein, this embodiment allows to pinpoint protein/single-stranded nucleic acid binding sites. Generally, it is the most 5' nucleotide on the positive strand and/or the most 3' nucleotide on the negative strand which allow determining of the binding site.

In a fourth aspect, the present invention provides a kit comprising (a) an oligonucleotide in accordance with the first aspect; (b) one or more enzymes selected from a ligase, a reverse transcriptase, a CircLigase, and a DNA polymerase; and (c) optionally a manual containing instructions for performing the method in accordance with the third aspect.

The Figures show:
- **Figure 1:**: Schematic representation of an exemplary oligonucleotide of the invention.
- **Figure 2:**: Implementation with Illumina-compatible sequences. (A) Exemplary oligonucleotide. (B) Illustration of workflow.

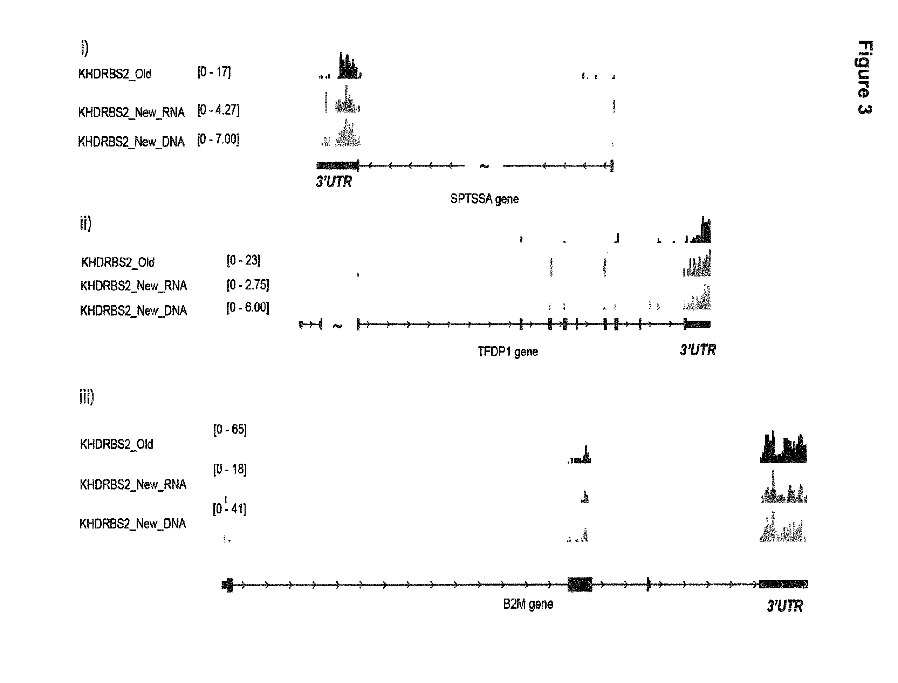

- **Figure 3:**: Example of RNA-binding profiles after Illumina sequencing. IGV (Integrative Genomics Viewer; distributed by Broad Institute) screenshots showing the distribution of cross-linking events on three example genes. KHDRBS2_old: uvCLAP prior art method, does not use the oligonucleotide of the invention profile of KHDRBS2 protein. KHDRBS2_New_RNA: Method of the invention that uses an all-RNA oligonucleotide and RNaseH mediated phosphorylation of the 5'-end of the cDNA. KHDRBS2_New_DNA: Method of the invention that uses a single RNA moiety in the 5' overhang that is cleaved with NaOH after which the 5'-end of the cDNA is phosphorylated with T4 PNK. 5'-phosphorylation is necessary for the circularization step.

The Examples illustrate the invention.

### Example 1

### Exemplary protocol

The protocol given below uses an oligonucleotide of the invention, e.g. the oligonucleotide displayed in the Figures.
(1) Protein of interest is enriched with a tandem-affinity purification.
(2) Bound RNA is trimmed with an RNase enzyme. RNasel is used which digests all bonds and leaves a 2',3'-cyclic phosphate group at the 3'-end of RNAs.
(3) Certain RNase enzymes may leave a 2',3'-cyclic phosphate, a 3'-phosphate or a 2'-phosphate group at the 3'-end. This type of end is not suitable for ligation, and must be repaired. T4 PNK at low pH (6.0) may be used for this purpose.
(4) The single-oligo is then ligated to the trimmed and repaired RNA, e.g. on beads. T4 RNA Ligase 1 and 5'-phosphorylated oligos are used.
(5) Excess single-oligo is washed away. At this stage, if desired, differentially labeled samples through the internal index part (barcode) in the single-oligo can be mixed together, before reverse transcription. Depending on how the indices are designed, between two to hundreds of samples can be mixed together.
(6) If the single-oligo contains a 3'-phosphate group which inhibits self-ligation, it is removed before reverse transcription. T4 PNK at low pH (6.0) is used.
(7) Reverse transcriptase is added together with necessary reagents such as dNTPs etc. There is no need to add and anneal an oligo to prime the reverse transcription.
(8) After reverse transcription two things are done:
   a. Remove all RNA
   b. Provide single-stranded DNA that has a 5'-phosphate and 3'-hydroxyl (necessary for CircLigase reaction)

There are two ways to achieve that:
(9)
   a) If the single-stranded extension of the single-oligo is made entirely of RNA, then RNAseH is added to the sample and incubated at 37°C for 10-60 minutes. RNaseH is a processive RNase that degrades RNA in RNA/DNA hybrids, and leaves a 5'-phosphate at the 5'-end.
   b) Alternatively, the sample is treated with 0.1 N NaOH (pH >13) at 70°C for 10 minutes first (or similar conditions that break down RNA but leave DNA intact). This destroys all RNA in the sample and denatures dsDNA to give rise to ssDNA. However, the 5'-end of the DNA molecule is a hydroxyl. This group is then converted to a phosphate using T4 PNK at neutral or slightly basic pH (7-8) in the presence of ATP. The second way can be used on all types of extensions e.g. all-RNA, single-RNA, or anything in between as long as the first nucleotide just after the double-stranded portion is RNA.
(10) The reaction is cleaned-up with any method that removes small molecules and protein while retaining ssDNA. SPRI (AMPure or SILANE) beads or columns, or phenol -chloroform extraction followed by ethanol precipitation may be used.
(11) The purified, 5'-phosphate, 3'-hydroxyl ssDNA is circularized with CircLigase or CircLigasell.
(12) Circularized DNA - without further purification or linearization (the non-nucleic acid spacer makes this possible) - is then used directly as a template in a PCR reaction to generate the final sequencing library.

### Results

The determination of a binding site of an RNA-binding protein on its cognate mRNA is illustrated in Figure 3. 5' terminal nucleotides of the sequencing reads of the positive strand are indicative of a binding site. Similarly, 3' terminal nucleotides of the sequencing reads of the negative strand are indicative of a binding site. The height of these peaks in this figure corresponds to the number of sequencing reads which terminate at the given position.

## Claims

1. An oligonucleotide comprising
(a) a double-stranded portion, which double-stranded portion is DNA and 9 to 30 base pairs in length;
(b) a loop connecting the 3' end of the first strand of said double-stranded portion with the 5' end of the second strand of said double-stranded portion, said loop comprising, in 5' to 3' direction:
(ba) a first DNA portion which is 4 to 20 nucleotides in length;
(bb) a non-nucleic acid spacer which
(i) does not interfere with the formation of a stem-loop by said oligonucleotide; and
(ii) causes polymerases to cease;
and
(bc) a second DNA portion which is 4 to 20 nucleotides in length; wherein said first DNA portion and said second DNA portion are not complementary to each other;
(c) a single-stranded overhang at its 5' end, said overhang being 5 to 40 nucleotides in length, wherein
(ca) the bond connecting said double-stranded portion with the nucleotide of said overhang which is directly adjacent to said double-stranded portion is cleavable under alkaline conditions; and
(cb) said overhang optionally comprises a barcode sequence, said barcode sequence preferably being 5 to 10 nucleotides in length;
and optionally
(d) within one, more or all of (a), (b) and (c), one, more or all of the following:
(da) one or more modified nucleotides;
(db) one or more sequences conferring compatibility with nucleic acid sequencing kits, such compatibility being preferably the presence of regions within said oligonucleotide which are complementary to primers comprised in said sequencing kits; and
(dc) one or more random bases.

2. The oligonucleotide of claim 1, wherein
(a) the melting temperature of said double-stranded portion is between 37°C and 70°C; and/or
(b) the number of mismatches between said first strand and said second strand is 0, 1, 2, or 3.

3. The oligonucleotide of claim 1 or 2, wherein said non-nucleic acid spacer is an oligo-ethyleneglycol spacer, preferably -(CH₂CH₂O)ₙ-, n being an integer number between 3 and 10, preferably 6.

4. The oligonucleotide of any one of the preceding claims, wherein said nucleotide of said overhang which is directly adjacent to said double-stranded portion comprises ribose.

5. The oligonucleotide of any one of the preceding claims, wherein said overhang is RNA.

6. Use of the oligonucleotide as defined in any one of the preceding claims for the generation of a library, preferably a sequencing library, from single-stranded nucleic acid.

7. The use of claim 7, wherein said generation involves reverse transcription and amplification.

8. The use of claim 6 or 7, wherein said oligonucleotide and primers for said amplification are the only oligonucleotides to be used.

9. A method of producing a library, preferably a sequencing library, from single-stranded RNA and/or single-stranded DNA, said method comprising or consisting of:
(a) ligating said single-stranded RNA and/or single-stranded DNA to the oligonucleotide as defined in any one of claims 1 to 7;
(b) reverse transcribing or transcribing, respectively, said single-stranded RNA or single-stranded DNA;
(c) hydrolyzing any RNA;
(d) circularizing the remaining DNA; and
(e) subjecting the product of (d) to amplification by PCR.

10. The method of claim 9, comprising one or more of the following further steps:
(aa) removing excess oligonucleotide after step (a) and prior to step (b);
(ab) pooling of different samples containing ligation products obtained in (a), wherein ligation products originating from different samples are barcoded differently in accordance with claim 1(c)(cb), and wherein said step (ab) to be effected after step (a) and prior to step (b);
(ac) removing, after step (a) and prior to step (b), the 3' phosphate from said ligation products to the extent it is present; and
(ca) removing protein and small molecules from single-stranded DNA obtained in (c), step (ca) to be effected after step (c) and prior to step (d).

11. The method of claim 9 or 10, wherein hydrolyzing any RNA is effected,
(c1) if the overhang of said oligonucleotide is RNA, by adding RNaseH; or
(c2) if said overhang contains DNA, by adding 0.1 M NaOH.

12. The method of any one of claims 9 to 11, comprising the following steps prior to step (a):
(1) optionally cross-linking a sample comprising cells;
(2) purifying from said sample a given protein, said protein being bound or cross-linked to single-stranded or double-stranded nucleic acid; and
(3) converting said nucleic acid, if it is double-stranded, into single-stranded nucleic acid; and
(4) trimming of the single-stranded nucleic acid bound or cross-linked to said protein.

13. The method of claim 12, wherein
(1) cross-linking is effected by
(1.1) UV-C;
(1.2) UV-A after feeding said cells 4-thiouridine; or
(1.3) formaldehyde;
(2) purifying is effected by using
(2.1) tandem affinity purification, in which case cross-linking is compulsory;
(2.2) an antibody specific for said protein;
and/or
(3) trimming of said single-stranded nucleic acid, to the extent it is RNA, is effected with an RNase and/or by sonication.

14. The method of claim 12 or 13, comprising one or more the following further steps after step (e):
(f) sequencing one, more or all members of the library obtained in step (e); and
(g) determining the most 5' nucleotide and/or the most 3' nucleotide of said nucleic acid bound or cross-linked to said protein, thereby determining the binding site of said protein on said nucleic acid.

15. A kit comprising
(a) an oligonucleotide as defined in any one of claims 1 to 5;
(b) one or more enzymes selected from a ligase, a reverse transcriptase, a CircLigase, and a DNA polymerase; and
(c) optionally a manual containing instructions for performing the method as defined in any one of claims 9 to 14.


SEQUENCE LISTING

<110> Max-Planck-Gesellschaft zur Förderung der Wissenschaften e.V.

<120> Cloning of single-stranded RNA

<130> Y1983 EP

<160> 3

<170> BiSSAP 1.3

<210> 1
<211> 50
<212> DNA
<213> Artificial Sequence

<220>
<223> Oligonucleotide of the invention. Between positions 31 and 32 there is
a non-nucleic acid spacer, preferably a hexa-ethyleneglycol moiety.

<220>
<221> variation
<222> 1,2,9,12
<223> /replace="a, c, g, t"

<220>
<221> variation
<222> 13
<223> /replace="a, c, g, u"

<400> 1
nntgtagcny ynnagatcgg aagagcgtcg tacgtgtgct cttccgatct          50


<210> 2
<211> 58
<212> DNA
<213> Artificial Sequence

<220>
<223> P5 primer

<400> 2
aatgatacgg cgaccaccga gatctacact ctttccctac acgacgctct tccgatct          58


<210> 3
<211> 64
<212> DNA
<213> Artificial Sequence

<220>
<223> P3 primer

<400> 3
caagcagaag acggcatacg agatcgtgat gtgactggag ttcagacgtg tgctcttccg          60

atct          64